(19) Europäisches Patentamt
European Patent Office
Office européen des brevets

(11) Veröffentlichungsnummer: **0 218 886**
**B1**

# (12) EUROPÄISCHE PATENTSCHRIFT

(45) Veröffentlichungstag der Patentschrift: **15.11.89**

(51) Int. Cl.⁴: **C07D 231/14**

(21) Anmeldenummer: **86112213.3**

(22) Anmeldetag: **04.09.86**

## (54) 1-Hydroxipyrazol-4-carbonsäure, Verfahren zu ihrer Herstellung und Verwendung.

(30) Priorität: **14.09.85 DE 3532879**

(43) Veröffentlichungstag der Anmeldung: **22.04.87 Patentblatt 87/17**

(45) Bekanntmachung des Hinweises auf die Patenterteilung: **15.11.89 Patentblatt 89/46**

(84) Benannte Vertragsstaaten: **AT BE CH DE FR GB IT LI NL SE**

(56) Entgegenhaltungen: **EP-A- 0 046 193**

(73) Patentinhaber: **BASF Aktiengesellschaft, Carl-Bosch-Strasse 38, D-6700 Ludwigshafen(DE)**

(72) Erfinder: **Boehm, Heinrich, Dr., Keplerweg 2, D-6708 Neuhofen(DE)**
Erfinder: **Rieber, Norbert, Dr., Liebfrauenstrasse 1c, D-6800 Mannheim 51(DE)**

EP 0 218 886 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 1-Hydroxipyrazol-4-carbonsäure, ihre Herstellung und Verwendung zur Herstellung pharmakologisch wertvoller Verbindungen.

Aus der Literatur (H. Kolbe, Liebigs Ann. Chem. 113 (1960) 125) ist die Umsetzung von Phenolen mit $CO_2$ in Gegenwart von Alkalicarbonaten zu Phenolcarbonsäuren bekannt. Entsprechend entsteht aus Kaliumpyrrolid mit $CO_2$ das Kaliumsalz der 2-Pyrrolcarbonsäure.

In der EP-B-0 046 193 ist die Herstellung von 1-Hydroxipyrazol beschrieben.

Es wurde nun überraschend gefunden, daß man 1-Hydroxipyrazol-4-carbonsäure vorteilhaft erhält, wenn man ein Alkali- oder Erdalkalisalz des 1-Hydroxipyrazols oder eine Mischung von 1-Hydroxipyrazol mit Alkali- oder Erdalkalicarbonaten bzw. -hydrogencarbonaten 1 bis 40 Stunden bei einem $CO_2$-Druck von 20 bis 300 bar auf 120 bis 250°C erhitzt.

Die Umsetzung kann z.B. durch folgendes Formelschema wiedergegeben werden:

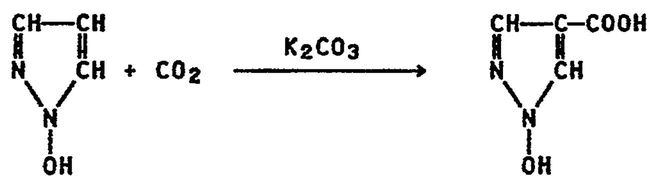


Die Umsetzung verläuft offensichtlich nicht analog der des Pyrrolids und war von jener nicht übertragbar, denn dort tritt die Carboxylgruppe in α, hier in β-Stellung zum Stickstoff ein.

Das erfindungsgemäße Verfahren liefert auf einfachem Weg die neue 1-Hydroxipyrazolverbindung. Diese findet als Ausgangsverbindung für pharmakologisch wertvolle Substanzen Verwendung (vgl. europäische Parallelanmeldung EP-A-0 215 380 ).

Als Alkali- und Erdalkali-1-Hydroxipyrazolide bzw. -carbonate und -hydrogencarbonate kommen die von Lithium, Natrium, Kalium, Magnesium und Calcium in Betracht. Bevorzugt werden Natrium und Kalium.

Die Reaktion kann wie folgt durchgeführt werden:

Ein Gemisch von 1-Hydroxipyrazol und mindestens äquivalenten Mengen Natrium- bzw. Kaliumcarbonat oder das Natrium- bzw. Kaliumsalz von 1-Hydroxipyrazol wird bei Abwesenheit oder in Gegenwart eines inerten Lösungsmittels 1 bis 40 Stunden bei einem $CO_2$-Druck von 20 bis 300 bar und 120 bis 250°C mit $CO_2$ umgesetzt. Nach Entspannen wird der Endstoff in üblicher Weise durch Ansäuern mit anschließendem Abfiltrieren oder Extrahieren abgetrennt und gegebenenfalls durch Umkristallisieren gereinigt. Als Lösungsmittel können polare protische und aprotische eingesetzt werden, z.B. Wasser, Alkohole, DMF, Acetonitril oder tertiäre Amine.

Die in dem folgenden Beispiel angeführten Teile bedeuten Gewichtsteile.

Beispiel

1 Teil 1-Hydroxipyrazol wird mit 4 Teilen Kaliumcarbonat intensiv gemischt und in einen Autoklaven gefüllt. Bei Raumtemperatur wird Kohlendioxid bis zu einem Druck von 50 bar aufgepreßt und dann 20 h auf 150°C erhitzt. Danach wird der Reaktorinhalt in Wasser gelöst, mit Salzsäure angesäuert, dadurch die 1-Hydroxipyrazol-4-carbonsäure ausgefällt, abfiltriert und aus Wasser umkristallisiert, Fp.: 230°C. Man erhält 0,68 Teile 1-Hydroxipyrazol-4-carbonsäure (45 % der Theorie).

## Patentansprüche

1. 1-Hydroxipyrazol-4-carbonsäure (I)

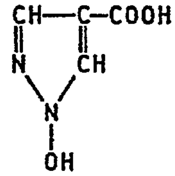

I

2. Verfahren zur Herstellung von 1-Hydroxipyrazol-4-carbonsäure, dadurch gekennzeichnet, daß man ein Alkali- oder Erdalkalisalz des 1-Hydroxipyrazols oder eine Mischung von 1-Hydroxipyrazol mit Alkali- oder Erdalkalicarbonaten bzw. -hydrogencarbonaten 1 bis 40 Stunden bei einem $CO_2$-Druck von 20 bis 300 bar auf 120 bis 250°C erhitzt.

3. Verfahren nach Anspruch 2, dadurch gekennzeichnet, daß die Reaktion in Gegenwart eines polaren Lösungsmittels durchgeführt wird.

4. Verwendung von 1-Hydroxipyrazol-4-carbonsäure der Formel (I) zur Herstellung pharmakologisch wertvoller Verbindungen.

## Claims

1. 1-Hydroxypyrazole-4-carboxylic acid (I)

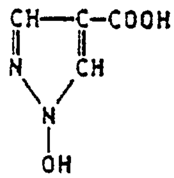

I

2. A process for the preparation of 1-hydroxypyrazole-4-carboxylic acid, wherein an alkali metal or alkaline earth metal salt of 1-hydroxypyrazole, or a mixture of 1-hydroxypyrazole with an alkali metal or alkaline earth metal carbonate or bicarbonate, is heated at from 120 to 250°C for from 1 to 40 hours under a $CO_2$ pressure of 20–300 bar.

3. A process as claimed in claim 2, wherein the reaction is carried out in the presence of a polar solvent.

4. Use of 1-hydroxypyrazole-4-carboxylic acid of the formula (I) for the preparation of pharmacologically useful compounds.

## Revendications

1. Acide 1-hydroxypyrazol-4-carboxylique (I)

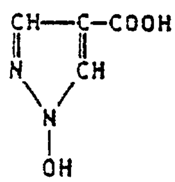

I

2. Procédé de préparation d'acide 1-hydroxypyrazol-4-carboxylique, caractérisé par le fait que l'on chauffe à une température de 120 à 250°C pendant 1 à 40 heures, sous une pression de $CO_2$ de 20 à 300 bar, un sel alcalin ou alcalino-terreux du 1-hydroxypyrazole ou un mélange de 1-hydroxypyrazole et de carbonates ou hydrogénocarbonates alcalins ou alcalino-terreux.

3. Procédé selon la revendication 2, caractérisé par le fait que la réaction est effectuée en présence d'un solvant polaire.

4. Utilisation de l'acide 1-hydroxypyrazol-4-carboxylique de formule I pour la préparation de composés précieux du point de vue pharmacologique.